# EUROPEAN PATENT APPLICATION

(11) **EP 1 316 328 A1**
(43) Date of publication of application: **04.06.2003**
(21) Application number: 01930032.6
(22) Date of filing: 10.05.2001
(51) Int. Cl.: A61M 29/02, A61F 2/06

(54) **MEDICAL TUBE-LIKE STENT**

(30) Priority: 15.05.2000 CN 00233798
(71) Applicant: Hocking, Donald Gordon, Tokyo 160-0022 (JP); IR Co., Ltd., Tokyo 171-0043 (JP); Cheng, Zheng Hoi, 518023 Shenzhen (CN)
(72) Inventor: CHENG, Zheng, Hoi, Shenzhen 518023 (CN)
(74) Representative: Barske, Heiko, Dr. rer. nat.
(86) International application number: JP0103904
(87) International publication number: WO01087401

(57) **Abstract**

The present invention provides a medical vascular stent that the support force in the diametrical direction is large, and the stent is hardly depressed during expansion in the diametrical direction, and the lumen wall is little stimulated during expansion, and the stent is not contracted in the axial direction. The medical vascular stent is a medical stent formed in a tubular shape by a metallic string net, and the metallic string net is structured so that a plurality of star-shaped cells 11 to 14 mutually connected in the circumferential direction are set as a net unit in the axial direction and a plurality of such units 15 to 21 are arranged in the axial direction, and the net units in the axial direction are mutually connected by connection of the respective star-shaped cells, constituting the net units in the neighboring axial directions, in the axial direction.

## Description

### Field of the Invention

The present invention relates to a medical vascular stent and in more particular to a medical vascular stent having a tubular structure formed with a metallic string net.

### Background of the Invention

A medical vascular stent has a tubular structure processed by metallic coils or metallic strings in a net shape. A stent of a metallic coil or string net tubular structure can be varied in diameter from a contraction state to an expansion state. Such a stent in a small-diameter state is inserted into a lumen at a morbid part in a tubular organ in the human body such as a bronchus, gullet, bile-duct, coronary artery, or other blood vessels and then expanded. By doing this, the lumen is expanded and improved in distribution, thus the purpose of treatment is accomplished.

Such a medical vascular stent is required to meet the conditions as indicated below.

Firstly, the support force inthediametricaldirection is so large that the stent is hardly depressed, with little stimulation against the lumen wall during expansion, and is not contracted in the axial direction during expansion in the diametrical direction.

Secondary, positioning of the stent is accurate and stable.

Thirdly, the stent is flexible and can be bent in the axial direction. Namely, when the stent is inserted into a bent lumen, it is required to be bent along the lumen.

Fourthly, the stent is free of projections toward and away from the wall and is flat so that it can be inserted into the lumen smoothly.

Fifthly, when being fixed and expanded inside the lumen, the stent is free of internal depressions or projections so that internal fluid flows smoothly.

However, a conventional medical vascular stent generally used is composed of a metallic net having a diamond or hexagonal structure. In a stent having the net structure or shape, connecting parts of the diamond or hexagon net moves in the axial direction of the vascular stent during expansion of the diameter thereof. Due to the movement of the parts of stent in the axial direction, an axial length of the stent is changed losing accuracy in dimension. As a result, not only an insertion position is moved but also there is a fear that depressions or projections may be formed on the outer surface of the vascular stent, which may stimulate the lumen wall and may damage it.

### Summary of the Invention

Therefore, an object of the present invention is to provide a medical vascular stent that the support force in the diametrical direction is larger and stabler so that local depressions are hardly formed.

Another object of the present invention is to provide a medical vascular stent that the inner and outer surfaces are flat, that the moving force to a lumen wall during expansion is small and that the axial length is not substantially changed before and after its expansion when the stent is be bent during its use.

The medical vascular stent according to the present invention is a medical stent formed in a tubular shape with a metallic string net, wherein the metallic string net has a structure that a plurality of star-shaped cells are interconnected along a circumferential direction forming a axial net unit in the string net, and that a plurality of such axial net units are arranged in the axial direction, wherein the axial net units are mutually interconnected with each other along an axial directions of the stent by connecting the respective star-shaped cells with each other included in the neighboring axial net units.

Further, in the medical vascular stent according to the present invention, the star-shaped cells have a hexagonal shape formed with an upper projection, left and right horizontal projections, and left and right lower projections, wherein they are symmetrical on the vertical axis and asymmetrical on the horizontal axis, wherein the plurality of star-shaped cells included in the respective axial net units in the axial direction are arranged so that the vertical directions are equally aligned, and wherein the plurality of star-shaped cells included in the neighboring axial net units in the axial directions are arranged so that the vertical directions are reversed.

Furthermore, in the medical vascular stent according to the present invention, the left and right projections and left and right lower projections of the star-shaped cell form a sine wave at each side of the cell and the lower side of the cell is formed by the upper projection of another cell, which is adjacently located to the cell.

Furthermore, in the medical vascular stent according to the present invention, the respective projections of said star-shaped cells are in a U shape or V shape formed by a smooth curve.

Further, the medical vascular stent according to the present invention is formed by a metallic string net in a tubular shape, in which the metallic string net has a plurality of metallic strings in a substantially sine wave-form arranged in parallel in the axial direction and extended in the circumferential direction of the tube and in which aplurality of curved metallic strings with a single peak arranged in the circumferential direction of the tube so as to mutually interconnect the neighboring sine wave-form metallic strings at neutral points of wave lengths forming the sine waves, wherein the curved metallic strings with a single peak are arranged so that the directions of the peaks of the neighboring curved metallic strings in the axial direction are opposite to each other.

Furthermore, in the medical vascular stent according to the present invention, the plurality of metallic strings in a substantially sine wave-form which are arranged in parallel in the axial direction are arranged so that the phases of the sine waves constituting the neighboring sine wave-form metallic strings are substantially the same in the circumferential direction of the tube.

Furthermore, in the medical vascular stent according to the present invention, the medical stent formed by the metallic string net in a tubular shape is characterized in that the wave length of the sine wave-form metallic strings is expanded and the amplitude thereof is contracted from an original state when the diameter of the stent is enlarged.

Furthermore, in the medical vascular stent according to the present invention, it is characterized in that each neutral point of the sine wave-form metallic strings is held in a substantially same position in the axial direction of the vascular stent before and after the enlargement of the diameter of the vascular stent.

Furthermore, in the medical vascular stent according to the present invention, it is characterized in that the metallic strings are a stainless steel or Ti-Ni series shape memory alloy.

### Brief Description of the Drawings

Fig. 1 is a plan development elevation view showing a structure of a metallic string net constituting a medical vascular stent according to the present invention before it is expanded,
Fig. 2 is a plan development elevation view showing the structure of a metallic string net constituting the medical vascular stent according to the present invention after it is expanded,

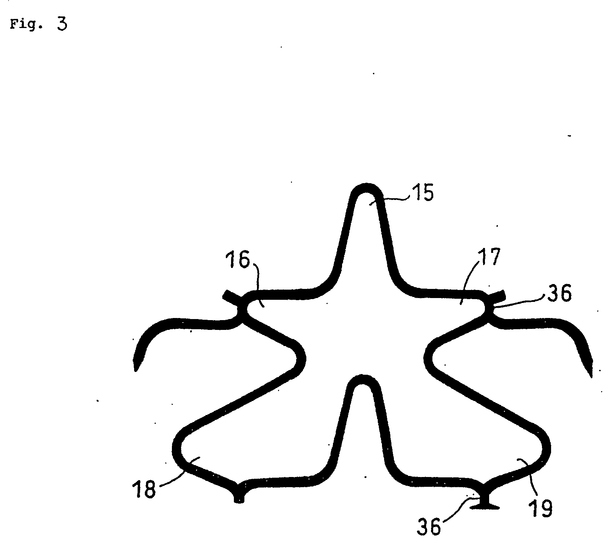

Fig. 3 is a plan view showing an enlarged one of the star-shaped cells shown in Fig. 2, and
Fig. 4 is a side view of a vascular stent composed of the metallic string net before it is expanded shown in Fig. 1.

### Detailed Description of the Invention

The embodiment of the present invention will be explained hereunder with reference to the accompanying drawings.

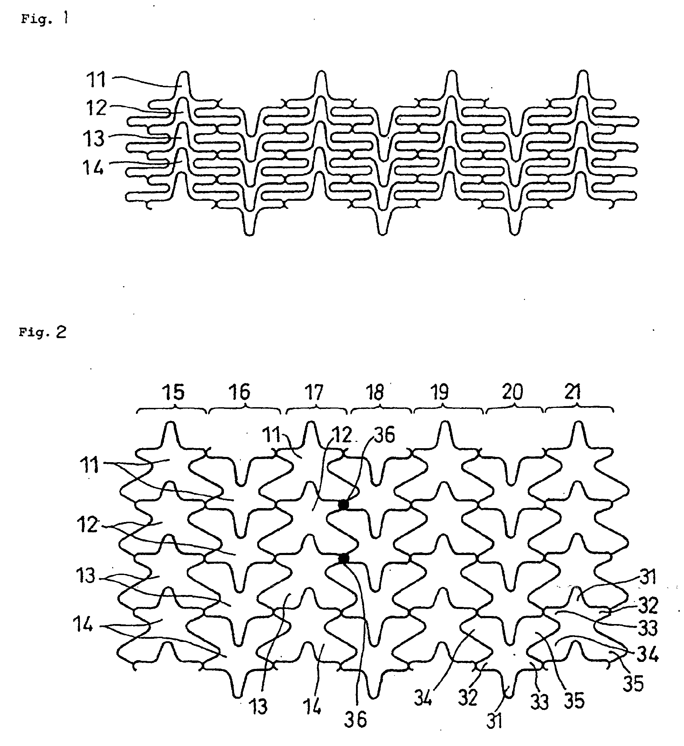

Fig. 1 is a plan development elevation view showing a structure of a metallic string net constituting a medical vascular stent of the present invention before expansion. And, Fig. 2 is a plan development elevation view showing the structure of a metallic string net constituting the medical vascular stent of the present invention after expansion. In the drawings, the transverse direction corresponds to the axial direction of the vascular stent and the vertical direction corresponds to the circumferential direction.

As shown in Figs. 1 and 2, in the metallic string net constituting the medical vascular stent of the present invention, an axial net unit 15 is composed of four star-shaped cells 11, 12, 13, and 14 of a substantially identical shape interconnected with each other in the circumferential direction. Seven axial net units 15 to 21 are arranged in the axial direction. The star-shaped cells 11 to 14, in the expanded state, as shown in Fig. 2, are composed of an upper projection 31, left and right horizontal projections 32 and 33, and left and right lower projections 34 and 35. The cells are of substantially hexagonal star-shape and are symmetrical on the vertical axis and asymmetrical on the horizontal axis. These projections are formed by a smooth U-shaped or V-shaped curve, and the left and right projections 34 and 35 are extended in the left and right horizontal direction in the non-expansion state shown in Fig. 1, while in the expansion state shown in Fig. 2, they are respectively extended slantwise downward.

In the star-shaped cells 11 to 14 included in the axial net unit 15, all the upper projections 31 thereof are directed upward. On the other hand, in the star-shaped cells 11 to 14 included in the axial net unit 16 adjacent to the unit 15 in the axial direction, all the upper projections 31 thereof are directed downward. Further, in the star-shaped cells 11 to 14 included in the third axial net unit 17, all the upper projections 31 thereof are directed upward in the same way as with the first unit 15. The star-shaped cells in the fourth to seventh axial net units 18 to 21 are arranged in the same way as the first to third units, in which the vertical directions of the cells included in the neighboring axial net units are alternately reversed.

The star-shaped cells 11 to 14 included in the first and second axial net units 15 and 16 are mutually connected with the horizontal projections 32 and 33 thereof. The star-shaped cells 11 to 14 included in the second and third axial net units 16 and 17 are also mutually connected with the horizontal projections 32 and 33 thereof. In the same way, the third to seventh axial net units 17 to 21 are also mutually connected with the horizontal projections 32 and 33 thereof.

The metallic string net constituting the medical vascular stent according to the present invention thus constructed has a characteristic that contours of both sides of the star-shaped cells 11 to 14 in the metallic string net form a sine wave extended in the circumferential direction of the vascular stent. The metallic string net according to the present invention has another characteristic that the top portions of the horizontal projections 32 and 33 constituting the star-shaped cells 11 to 14 in any one of the axial net units are connected to the top portions of the horizontal projections 32 and 33 constituting the star-shaped cells 11 to 14 in the adjacent axial net units at the neutral points 36 of the wave lengths forming the sine waves, as shown in Figs. 1 and 2. Observing the upper projection 31 and the horizontal projections 32 and 33 constituting each of the star-shaped cells 11 to 14 as a whole, these projections form a single peak of the upper projections 31 with its ridgelines gently extend horizontally to the right and left side projections 32 and 33. Therefore, the metallic strings having the sine wave-form are mutually connected by the curved metallic strings with a single peak at the neutral points 36 of the wave lengths forming the sine waves. These characteristics may be applied to the star-shaped cells 11 to 14 belonging to any of the axial net units. //For example, in the first and second axial net unit 15 and 16, the vertical directions of the star-shaped cells 11 to 14 are opposite with each other. The metallic string nets forming the contours of both sides of the star-shaped cells 11 to 14 belonging to the second axial net unit 16 have the substantially sine wave-form extending in the circumferential direction of the vascular stent. Further, the sine wave-form metallic strings are mutually connected by the curved metallic strings with a single peak extended downward at the neutral points 36 of the wave lengths forming the sine waves.

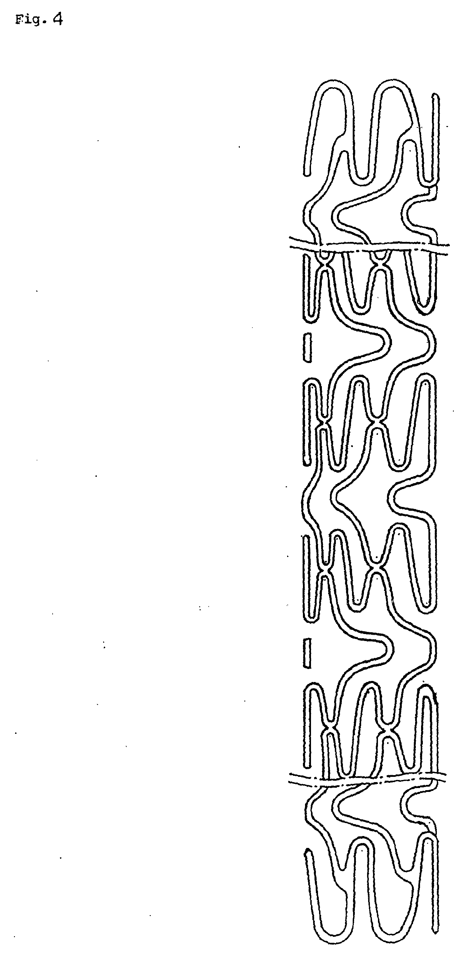

Fig. 4 is a side view of a vascular stent formed by the metallic string net before expansion as shown in Fig. 1. In the drawing, the same symbols are assigned to the same components as those shown in Figs. 1 and 2. The stent in this embodiment is 12 to 13 mm in length and about 1 mm in diameter. The vascular stent may be manufactured by preparing a flat metallic string net by processing a flat metallic plate or ceramics plate mechanically or scientifically and then bending it in a tubular shape or by processing a tubular metallic plate or ceramics plate in a metallic string net shape by processing it mechanically or chemically.

Further, the number of star-shaped cells constituting the vascular stent is not limited to the aforementioned embodiment. For example, the number of cells in the axial direction (the number of axial net units in the axial direction) and the size of cells are selected according to the morbid length. Further, the number and size of cells in the circumferential direction are selected according to the size of a morbid lumen.

The vascular stent having such a constitution is mounted at the end of a catheter not shown in the drawing and inserted into a morbid lumen in the human body in a state that the diameter thereof is not expanded. The vascular stent is then expanded in diameter. Various methods are known for expansion of the diameter of the vascular stent. For example, air is fed into a tubular balloon inserted in a vascular stent mounted at the end of a catheter and the balloon can be inflated.

The medical vascular stent of the present invention can be easily bent in any direction from the axial direction when it is inserted into a bent morbid part due to the structure, in which the sine wave-form metallic strings arranged at predetermined intervals in the axial direction are mutually connected by the curved metallic strings with a single peak alternately changing the directions thereof vertically in the axial direction, as shown in Fig. 4.

Further, the medical vascular stent of the present invention neither causes the expansion or contraction of the vascular stent in the axial direction nor any change in the position thereof when the vascular stent is changed from the contracted state to the expanded state in the diametric direction, since the sine wave-form metallic strings arranged at predetermined intervals in the axial direction are mutually connected by the curved metallic strings with a single peak at the neutral points 36 of the wave lengths forming the sine waves thereof, which do not move in the axial direction while the stent is diametrically expanded. Thus, the stent according to the present invention will neither stimulate nor damage the lumen wall.

More specifically, in the medical vascular stent of the present invention, the wave length of the sine wave of the metallic strings are expanded and the amplitude thereof is contracted when the diameter of the stent is enlarged from original state where the diameter is not enlarged. However, the neutral points 36 of the sine wave of the metallic strings are kept in substantially the same axial positions of the vascular stent at times before and after the diameter of the vascular stent is enlarged.

Furthermore, according to the metallic string nets constituting the stent, large support force is realized in the circumferential direction and the inner and outer surfaces which are flat and have few local irregularities are structured, since curved metallic strings with a single peak mutually connect a plurality of sine wave-form metallic strings arranged at predetermined intervals in the axial direction at the neutral points 36 of the wave lengths.

Furthermore, in the metallic string nets constituting the medical vascular stent according to the present invention, the end portions of the vascular stent are terminated with closed metallic string loops so that no cut parts of the metallic strings appear there. The stent according to the present invention can be inserted into a morbid lumen without any resistance and any fear of causing damages to the inner wall of the lumen since the stent is composed of a plurality of sine waveform metallic strings, which are interconnected with the curved metallic strings with a single peak.

## Claims

1. A medical vascular stent formed in a tubular shape with a metallic string net, wherein the metallic string net has a structure that a plurality of star-shaped cells are interconnected along a circumferential direction forming a axial net unit in the string net, and that a plurality of such axial net units are arranged in the axial direction, wherein the axial net units are mutually interconnected with each other along an axial directions of the stent by connecting the respective star-shaped cells with each other included in the neighboring axial net units.

2. A medical vascular stent according to Claim 1, wherein said star-shaped cells have a hexagonal shape formed with an upper projection, left and right horizontal projections, and left and right lower projections, wherein they are symmetrical on the vertical axis and asymmetrical on the horizontal axis, wherein said plurality of star-shaped cells included in the respective axial net units in the axial direction are arranged so that the vertical directions are equally aligned, and wherein said plurality of star-shaped cells included in the neighboring axial net units in the axial directions are arranged so that the vertical directions are reversed.

3. A medical vascular stent according to Claim 2, wherein said left and right horizontal projections and said left and right lower projections of said star-shaped cell form a sine wave at each side of the cell and the lower side of said cell is formed by said upper projection.

4. A medical vascular stent according to Claim 3, wherein said respective projections of said star-shaped cells are in a U shape or V shape formed by a smooth curve.

5. Amedical vascular stent formed by a metallic string net in a tubular shape, wherein said metallic string net has a plurality of metallic strings in a substantially sine wave-form arranged in parallel in the axial direction and extended in the circumferential direction of said tube and in which a plurality of curved metallic strings with a single peak arranged in the circumferential direction of said tube so as to mutually interconnect said neighboring sine wave-form metallic strings at neutral points of wave lengths forming said sine waves, wherein said curved metallic strings with a single peak are arranged so that the directions of the peaks of said neighboring curved metallic strings in the axial direction are opposite to each other.

6. A medical vascular stent according to Claim 5, wherein said plurality of metallic strings in a substantially sine wave-form which are arranged in parallel in the axial direction are arranged so that the phases of said sine waves constituting the neighboring sine wave-form metallic strings are substantially the same in the circumferential direction of the tube.

7. A medical vascular stent according to Claim 6, wherein the wavelength of said sine waveform metallic strings is expanded and the amplitude thereof is contracted from an original state when the diameter of the stent is enlarged.

8. A medical vascular stent according to Claim 7, wherein said each neutral point of the sine wave-form metallic strings is held in a substantially same position in the axial direction of the vascular stent before and after the enlargement of the diameter of the vascular stent.

9. A medical vascular stent according to any of Claims 6, 7, and 8, wherein said metallic strings are a stainless steel or Ti-Ni series shape memory alloy.
